## Europäisches Patentamt

## European Patent Office

## Office européen des brevets

(11) Publication number: **0 211 020**
**B1**

## (12) EUROPEAN PATENT SPECIFICATION

(45) Date of publication of patent specification: **05.09.90**

(51) Int. Cl.⁵: **A 61 K 9/10, A 61 K 31/575**

(21) Application number: **86900748.4**

(22) Date of filing: **07.01.86**

(86) International application number:
**PCT/DK86/00002**

(87) International publication number:
**WO 86/03966 17.07.86 Gazette 86/17**

(54) OPHTHALMIC GEL COMPOSITION AND METHOD OF TREATING EYE INFECTIONS.

(30) Priority: **07.01.85 GB 8500310**

(43) Date of publication of application:
**25.02.87 Bulletin 87/09**

(45) Publication of the grant of the patent:
**05.09.90 Bulletin 90/36**

(84) Designated Contracting States:
**AT BE CH DE FR GB IT LI LU NL SE**

(56) References cited:
**EP-A-0 020 794     FR-A-2 407 214**
**DE-A-2 654 508     GB-A-2 013 084**
**DK-B- 135 267**

**Remingtons Pharmaceutical sciences, 1975, Easton Pennsylvania, pages 1537-38**

**Rote liste 1977/78 "Item 31005 B, Fucidine."**

**Brit. J. Ophthal Volume 53, No. 1, January 1969**

**A.J. Chadwich and B.Jackson "Intraocular penetration of the antibiotic Fucidin." See pages 26-29**

(73) Proprietor: **LEO PHARMACEUTICAL PRODUCTS LTD. A/S (LOVENS KEMISKE FABRIK PRODUKTIONSAKTIESELSKAB)**
**Industriparken 55**
**DK-2750 Ballerup (DK)**

(72) Inventor: **DELEURAN, Merete**
**Hoveltsbjergvej**
**DK-3450 Allerod (DK)**

(74) Representative: **Kinzebach, Werner, Dr. et al Patentanwälte Reitstötter, Kinzebach und Partner Sternwartstrasse 4 Postfach 86 06 49 D-8000 München 86 (DE)**

## Description

The present invention relates to an ophthalmic gel composition for human and veterinary use comprising an ophthalmic drug and a vehicle based on a polyanionic polymer.

One of the major problems of topical ophthalmic therapy is to maintain an adequate concentration of the ophthalmic drug at the desired site of action for a prolonged period of time. Thus, only a small volume of preparation can be contained in the fornix inferior and the preparation applied tends to be diluted by the tears and to be drained through the nasolacrimal duct.

It is well known that the duration of action of cationic ophthalmic drugs may be increased by incorporating such drugs into gels based on polyanionic polymers, cf. DE—OS 29 02 863, R. D. Schoenwald et al: Influence of high-viscosity vehicles on miotic effect of pilocarpine, Jour. of Pharm. Sciences, Vol. 67, No. 9, Sept. 1978, 1280—1283, F. Bottari et al: Semisolid ophthalmic vehicles II: Evaluation in albino rabbits of aqueous gel-type vehicles containing lidocaine and benzocaine, Can. J. of Pharm. Sci., vol. 14, No. 2 1979, 39—43, and Engelbert Graf et al: Interaction of Carbopol® 934, with diphenhydramine and dexchlorpheniramine, Acta Pharmaceutica Technologica, 29 (3), 1983, 209—215.

These gel compositions exhibit a duration of action which is about twice that of a conventional ophthalmic drug preparation.

Similarly, GB—A—2 013 084 discloses an aqueous gel for treating eye diseases which is a long-acting well-retained gel preparation containing an ophthalmic drug. The ophthalmic drug is reacted with a polymer to provide a complex and/or salt suitable for topical application. Thus, the ophthalmic drug is a cationic drug such as pilocarpine. The gel-forming agent is a high-molecular weight polymer having carboxylic or anhydride functional groups and is used in the gel compositions at a level of from about 2 to about 8% by weight.

DE—A—2 654 508 relates to fusidic acid and pharmaceutical compositions containing fusidic acid. Ophthalmic preparations are not mentioned.

Brit. J. Ophthal. (1969), 53, 26 relates to a scientific study concerning the degree of penetration of fusidic acid into the aqueous humour in the normal intact human eye after systemic administration in the form of capsules. Ophthalmic compositions are not disclosed.

Surprisingly, it has now been found that eye infections can be combatted very effectively with a composition comprising fusidic acid suspended in a gel of the above mentioned type.

The composition of the invention comprises from 0.1 to 4% w/v of fusidic acid suspended in an aqueous vehicle containing from 0.2 to 2% w/v of polyanionic polymer.

The surprising efficiency of the composition of the invention is evidenced by the fact that the duration of action is about 10 times that of a preparation based on fusidic acid and a conventional gelling agent, cf. the example below.

The very substantial prolongment of the action of fusidic acid is particularly surprising, since hitherto it was believed that the prolonged action of the prior art compositions is based on an interaction of a positively charged drug with the negatively charged polymer. Thus, it might be expected that a drug, such as fusidic acid, which is negatively charged at the pH value of the eye, i.e. about 7.4, would be unable to interact with the polyanionic polymer and to provide a prolonged action.

The composition of the invention is particularly useful for the local treatment of eye infections. Thus, whereas conventional ophthalmic preparations, such as chloroamphenicol eye drops, have to be applied 5—6 times daily or even more, it is sufficient to apply preparations based on the composition of the invention 1—2 times daily.

The composition of the invention preferably contains about 1% w/v of fusidic acid in the form of particles having a particle size not exceeding 10 μm and preferably between 2 and 5 μm.

A polyanionic polymer is preferably a carboxyvinyl polymer having a molecular weight of from 400.000 to 6 million. The viscous solutions which are formed during the preparation of the ophthalmic polymer suspension have a viscosity of from 10 to 20.000 mPas at 25°C measured on a RVT Brookfield Viscosimeter.

The polyanionic polymer is preferably of the type which is commercially available under the trade name "Carbopol"® (B. F. Goodrich Company). A particularly preferred polyanionic polymer is "Carbopol® 934". The "Carbopol® polymers do not blur the normal vision because the gel structure breaks down shortly after application to the eye under the influence of ions contained in the lacrymal fluid.

The pH value of the composition of the invention is preferably from 5.0 to 6.5 and more preferably about 5.8.

The adjustment of the pH value is preferably effected with a pharmaceutically and physiologically acceptable base, such as sodium hydroxide.

A preparation based on the composition of the invention may contain auxiliary agents, such as preservatives, stabilizing agents and bacteriostatic agents.

Preparations based on the composition of the invention are preferably used in dosages of from 5 to 100 mg and more preferably from 20 to 50 mg when the preparation is applied into the fornix inferior of an infected eye.

The frequency of dosing varies dependant upon the severity of the infection. However, as mentioned above an application twice a day ordinarily suffices.

The invention also relates to the use of an ophthalmic gel composition according to the present invention for preparing a pharmaceutical preparation for treating eye infections. Said eye infections are treated by applying an effective amount of a preparation based on the composition of the invention into the fornix inferior of the infected eye.

The invention will be described in detail with reference to the following non-limiting example:

Example

An eye preparation having the following composition per ml was prepared:

Preparation A:

| | |
|---|---|
| Fusidic acid, micronized, sterile | 10 mg |
| "Carbopol 934"® (Carboxy vinyl polymer) | 5 mg |
| Sodium hydroxide, 5N, q.s. for pH 5—6.0 | |
| Mannitol | 50 mg |
| Benzalkonium chloride | 0.1 mg |
| Tetracemin disodium (disodium dihydrogen edetate) | 0.5 mg |
| Water, sterile, to make | 1 ml |

Benzalkonium chloride, tetracemin disodium and mannitol are dissolved in sterile water.

The "Carbopol 934"® is suspended aseptically in the solution and sterilized by autoclaving at 120°C for 20 minutes. The suspension is cooled and the sterile fusidic acid is suspended therein. Finally, the suspension is neutralized by addition of sterile sodium hydroxide solution.

The preparation (in the following called Preparation A) thus prepared was compared with a preparation of fusidic acid suspended in a vehicle based on a conventional gel forming agent and further containing preservatives, tonicity agents and a buffer. This preparation (in the following called Preparation B) had the following composition per ml:

Preparation B:

| | |
|---|---|
| Fusidic acid, micronized, sterile | 10 mg |
| Hydroxyethylcellulose | 4 mg |
| Methyl cellulose | 0.2 mg |
| Sodium acetate | 1 mg |
| Glacial acetic acid, q.s. for pH 5—6.0 | |
| Sodium chloride | 9 mg |
| Phenethanol | 2.5 mg |
| Water, sterile, to make | 1 ml. |

The two preparations were compared in the following manner:

Five New Zealand white rabbits weighing about 3 kg were used in the studies. They were designated Nos. 1—5 and placed in restraining boxes during the first 6 hours of the investigation period and at the 24 hour sampling. Between the 6 hours and the 24 hour sampling, the animals were housed individually and given pelleted food and water ad libitum.

Samples for microbiologic determination were taken by placing 6.0 mm AA discs (Whatman) in the fornix inferior, closing the eye lids for approximately 10 seconds and removing the discs with forceps. The discs were placed on the surface of inoculated agar dishes, 14 cm in diameter, together with discs which has been pre-treated with standard solutions for 10 seconds.

The petri dishes were incubated for 16—18 hours at the optimum temperature for the test organisms in question. The inhibition zones of the samples were measured and interpolated on the standard curve which was established as the best response line from the inhibition zones for the standard solutions.

Prior to each investigation the lacrymal fluid of each rabbit was tested for the absence of substances which are inhibitory to the test organisms.

One drop of each preparation was instilled in the fornix inferior of the left eye of each of the five rabbits. Samples were taken at 1, 2, 4, 6 and 24 hours following treatment.

There was a period of at least 72 hours between treatments with different preparations.

The results are presented in the table which shows that fusidic acid is present in measurable concentrations 24 hours after the treatment.

However, the concentration of fusidic acid in the 24 hour samples is approximately ten times higher for preparation A than for preparation B.

TABLE

| | Animal No. | μg fusidic acid per ml lacrymal fluid | | | | | |
|---|---|---|---|---|---|---|---|
| | | Hours after treatment | | | | | |
| | | 0 | 1 | 2 | 4 | 6 | 24 |
| Preparation A | 1 | — | 45 | 45 | 25 | 20 | 6 |
| | 2 | — | 32 | 20 | 6 | 5 | 4 |
| | 3 | — | 113 | 24 | 20 | 4 | 5 |
| | 4 | — | 32 | 28 | 40 | 22 | 4 |
| | 5 | — | 57 | 18 | 6 | 15 | 4 |
| Preparation B | 1 | — | 14 | 5 | 5 | 3 | 0.4 |
| | 2 | — | 20 | 12 | 11 | 8 | 0.2 |
| | 3 | — | 28 | 28 | 8 | 6 | 0.6 |
| | 4 | — | 23 | 10 | 4 | 4 | 0.4 |
| | 5 | — | 34 | 12 | 10 | 4 | 0.5 |

—: indicates a value lower than the detection limit of 0.02 μg fusidic acid per ml.

**Claims for the Contracting States: BE CH DE FR GB IT LI LU NL SE**

1. An ophthalmic gel composition for human and veterinary use comprising an ophthalmic drug and a vehicle based on a polyanionic polymer, said composition comprising 0.1 to 4% w/v of fusidic acid suspended in an aqueous vehicle containing from 0.2 to 2% w/v of polyanionic polymer.

2. An ophthalmic gel composition according to claim 1, said composition comprising about 1% w/v of fusidic acid in the form of particles having a particle size not exceeding 10 μm.

3. An ophthalmic gel composition according to claim 2, wherein the particle size of the suspended particles of fusidic acid is between 2 and 5 μm.

4. An ophthalmic gel composition according to claim 1, said composition having a pH value adjusted to from 5.0 to 6.5.

5. An ophthalmic preparation comprising a gel composition according to claim 1 and an auxiliary agent selected from preservatives, stabilizers and bacteriostatic agents.

6. An ophthalmic preparation according to claim 5, said preparation comprising

| | |
|---|---|
| Fusidic acid | 1% w/v |
| Carboxyvinyl polymer | 0.5% w/v |
| Mannitol | 5% w/v |
| Benzalkonium chloride | 0.01% w/v |
| Disodium dihydrogen edetate | 0.05% w/v |

said preparation having a pH value adjusted to 5.8.

7. The use of the ophthalmic gel compositions of claims 1 to 4 for preparing an ophthalmic preparation for treating eye infections.

# EP 0 211 020 B1

## Claims for the Contracting State: AT

1. A method for preparing an ophthalmic gel composition for human and veterinary use which comprises providing 0.1 to 4% w/v of fusidic acid as an ophthalmic drug suspended in an aqueous vehicle containing from 0.2 to 2% w/v of polyanionic polymer.

2. The method of claim 1, wherein said composition comprises about 1% w/v of fusidic acid in the form of particles having a particle size not exceeding 10 μm.

3. The method of claim 2, wherein the particle size of the suspended particles of fusidic acid is between 2 and 5 μm.

4. The method of claim 1, wherein said composition has a pH value adjusted to from 5.0 to 6.5.

5. A method for preparing an ophthalmic preparation which comprises the admixture of a gel composition according to claim 1 and an auxiliary agent selected from preservatives, stabilizers and bacteriostatic agents.

6. The method of claim 5, wherein a preparation is obtained comprising

| | |
|---|---|
| Fusidic acid | 1% w/v |
| Carboxyvinyl polymer | 0.5% w/v |
| Mannitol | 5% w/v |
| Benzalkonium chloride | 0.01% w/v |
| Disodium dihydrogen edetate | 0.05% w/v |

said preparation having a pH value adjusted to 5.8.

7. The use of the ophthalmic gel compositions of claims 1 to 4 for preparing an ophthalmic preparation for treating eye infections.

## Patentansprüche für die Vertragsstaaten: BE CH DE FR GB IT LI LU NL SE

1. Ophthalmische Gelzusammensetzung zur Verwendung bei Mensch und Tier, umfassend ein ophthalmisches Medikament und einen Träger, basierend auf einem polyanionischen Polymer, wobei die Zusammensetzung 0.1 bis 4% G/V Fusidinsäure, suspendiert in einem wässrigen Träger umfaßt, der 0.2 bis 2% G/V des polyanionischen Polymers enthält.

2. Ophthalmische Gelzusammensetzung nach Anspruch 1, worin die Zusammensetzung etwa 1% G/V Fusidinsäure in Form von Partikeln umfaßt, die nicht größer sind als 10 μm.

3. Ophthalmische Gelzusammensetzung nach Anspruch 2, wobei die Partikelgröße der suspendierten Fusidinsäurepartikel zwischen 2 und 5 μm liegt.

4. Ophthalmische Gelzusammensetzung nach Anspruch 1, deren pH-Wert auf 5.0 bis 6.5 eingestellt ist.

5. Ophthalmisches Präparat, umfassend eine Gelzusammensetzung nach Anspruch 1 und ein Hilfsmittel, ausgewählt unter Konservierungsmitteln, Stabilisatoren und bakteriostatischen Mitteln.

6. Ophthalmisches Präparat nach Anspruch 5, umfassend

| | |
|---|---|
| Fusidinsäure | 1% G/V |
| Carboxyvinylpolymer | 0.5% G/V |
| Mannitol | 5% G/V |
| Benzalkoniumchlorid | 0.01% G/V |
| Dinatrium-dihydrogen-edetat | 0.05% G/V |

wobei der pH-Wert des Präparats auf 5.8 eingestellt ist.

7. Verwendung der ophthalmischen Gelzusammensetzung nach den Ansprüchen 1 bis 4 zur Herstellung eines ophthalmischen Präparats zur Behandlung von Augenentzündungen.

## Patentansprüche für den Vertragsstaat: AT

1. Verfahren zur Herstellung einer ophthalmischen Gelzusammensetzung zur Verwendung bei Mensch und Tier, wobei man 0.1 bis 4% G/V Fusidinsäure als ophthalmisches Medikament suspendiert in einem wässrigen Träger bereitstellt, der 0.2 bis 2% G/V eines polyanionischen Polymers enthält.

2. Verfahren nach Anspruch 1, worin die Zusammensetzung etwa 1% G/V Fusidinsäure in Form von Partikeln umfaßt, die nicht größer sind als 10 μm.

3. Verfahren nach Anspruch 2, wobei die Partikelgröße der suspendierten Fusidinsäurepartikel zwischen 2 und 5 µm liegt.

4. Verfahren nach Anspruch 1, wobei der pH-Wert der Zusammensetzung auf 5.0 bis 6.5 eingestellt ist.

5. Verfahren zur Herstellung eines ophthalmischen Präparats, wobei man eine Gelzusammensetzung nach Anspruch 1 und ein Hilfsmittel, ausgewählt unter Konservierungsmitteln, Stabilisatoren und bakteriostatischen Mitteln, vermischt.

6. Verfahren nach Anspruch 5, wobei man ein Präparat erhält, das umfaßt:

| | |
|---|---|
| Fusidinsäure | 1% G/V |
| Carboxyvinylpolymer | 0.5% G/V |
| Mannitol | 5% G/V |
| Benzalkoniumchlorid | 0.01% G/V |
| Dinatrium-dihydrogen-edetat | 0.05% G/V |

wobei der pH-Wert des Präparats auf 5.8 eingestellt ist.

7. Verwendung der ophthalmischen Gelzusammensetzung nach den Ansprüchen 1 bis 4 zur Herstellung eines ophthalmischen Präparats zur Behandlung von Augenentzündungen.

**Revendications pour les Etats contractants: BE CH DE FR GB IT LI LU NL SE**

1. Une composition de gel ophtalmique pour l'utilisation humaine et vétérinaire comprenant un médicament ophtalmique et un véhicule à base d'une polymère polyanionique, ladite composition comprenant 0,1 à 4% en poids/volume d'acide fusidique en suspension dans un véhicule aqueux contenant de 0,2 à 2% en poids/volume de polymère polyanionique.

2. Une composition de gel ophthalmique selon la revendication 1, ladite composition comprenant environ 1% en poids/volume d'acide fusidique sous la forme de particules ayant une taille de particule ne dépassant 10 µm.

3. Une composition de gel ophtalmique selon la revendication 2, selon laquelle la taille de particule des particules en suspension d'acide fusidique est comprise entre 2 et 5 µm.

4. Une composition de gel ophtalmique selon la revendication 1, ladite composition ayant une valeur de pH ajustée à 5,0 à 6,5.

5. Une préparation ophtalmique comprenant une composition de gel selon la revendication 1 et un agent auxiliaire choisi parmi les conservateurs, les stabilisants et des agents bactériostatiques.

6. Une préparation ophtalmique selon la revendication 5, ladite préparation comprenant:

| | |
|---|---|
| Acide fusidique | 1% en poids/volume |
| Polymère carboxyvinylique | 0,5% en poids/volume |
| Mannitol | 5% en poids/volume |
| Chlorure de benzalkonium | 0,01% en poids/volume |
| Dihydrogéno édétate disodique | 0,05% en poids/volume |

ladite préparation ayant une valeur de pH ajustée à 5,8.

7. L'utilisation des compositions de gel ophtalmique des revendications 1 à 4 pour la préparation d'une préparation ophtalmique pour traiter les infections des yeux.

**Revendications pour l'Etat contractant: AT**

1. Un procédé de préparation d'une composition de gel ophtalmique pour l'utilisation humaine et vétérinaire qui consiste à fournir 0,1 à 4% en poids/volume d'acide fusidique en tant que médicament ophtalmique en suspension dans un véhicule aqueux contenant de 0,2 à 2% en poids/volume de polymère polyanionique.

2. Le procédé selon la revendication 1, selon lequel ladite composition comprend environ 1% en poids/volume d'acide fusidique sous la forme de particules ayant une taille de particule ne dépassant 10 µm.

3. Le procédé selon la revendication 2, selon lequel la taille de particule des particules en suspension d'acide fusidique est comprise entre 2 et 5 µm.

6

4. Le procédé selon la revendication 1, selon lequel ladite composition a une valeur de pH ajustée à 5,0 à 6,5.

5. Un procédé de préparation d'une préparation ophtalmique qui comprend le mélangeage d'une composition de gel selon la revendication 1 et d'un agent auxiliaire choisi parmi les conservateurs, les stabilisants et les agents bactériostatiques.

6. Le procédé selon la revendication 5, selon lequel on obtient une préparation comprenant:

| | |
|---|---|
| Acide fusidique | 1% en poids/volume |
| Polymère carboxyvinylique | 0,5% en poids/volume |
| Mannitol | 5% en poids/volume |
| Chlorure de benzalkonium | 0,01% en poids/volume |
| Dihydrogéno édétate disodique | 0,05% en poids/volume |

ladite préparation ayant une valeur de pH ajustée à 5,8.

7. L'utilisation des compositions de gel ophtalmique des revendications 1 à 4 pour la préparation d'une préparation ophtalmique pour traitement des infections des yeux.